(19) Europäisches Patentamt
European Patent Office
Office européen des brevets

(11) **EP 2 988 675 B1**

(12) **EUROPEAN PATENT SPECIFICATION**

(45) Date of publication and mention
of the grant of the patent:
**30.08.2023 Bulletin 2023/35**

(51) International Patent Classification (IPC):
*A61B 8/00* (2006.01)    *A61B 8/08* (2006.01)
*G06T 7/00* (2017.01)    *G06T 7/20* (2017.01)
*G06T 7/269* (2017.01)    *G16H 50/30* (2018.01)

(21) Application number: **14707245.8**

(22) Date of filing: **13.02.2014**

(52) Cooperative Patent Classification (CPC):
**A61B 8/08; A61B 8/4477; A61B 8/5223;
A61B 8/5246; G06T 7/0016; G06T 7/269;
G16H 50/30;** A61B 8/4209; A61B 8/485;
G06T 2207/10132; G06T 2207/30004

(86) International application number:
**PCT/IB2014/058966**

(87) International publication number:
**WO 2014/174379 (30.10.2014 Gazette 2014/44)**

(54) **METHOD AND DEVICE FOR QUANTITATIVE DYNAMIC EVALUATION OF SKELETAL MUSCLES FUNCTIONALITY**

VERFAHREN UND VORRICHTUNG ZUR QUANTITATIVEN DYNAMISCHEN AUSWERTUNG DER FUNKTIONALITÄT VON SKELETTMUSKELN

PROCÉDÉ ET DISPOSITIF POUR UNE ÉVALUATION QUANTITATIVE DYNAMIQUE DE LA FONCTIONNALITÉ DES MUSCLES SQUELETTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2013 IT AQ20130003**

(43) Date of publication of application:
**02.03.2016 Bulletin 2016/09**

(73) Proprietors:
• **Amid s.r.l.**
**67039 Sulmona (AQ) (IT)**
• **Fondazione Apostolo**
**20121 Milano (IT)**

(72) Inventors:
• **BERTELÈ, Laura**
**I-23807 Merate (LC) (IT)**
• **APOSTOLO, Carlo**
**I-23807 Merate (LC) (IT)**
• **PEDRIZZETTI, Gianni**
**I-59100 Prato (PO) (IT)**
• **TONTI, Gianni**
**I-67039 Sulmona (AQ) (IT)**

(74) Representative: **V.O.**
**P.O. Box 87930**
**2508 DH Den Haag (NL)**

(56) References cited:
EP-A1- 2 347 716        WO-A1-2012/009023
US-A1- 2012 203 107

• **JUN SHI ET AL: "Recognition of Finger Flexion from Ultrasound Image with Optical Flow: A Preliminary Study", BIOMEDICAL ENGINEERING AND COMPUTER SCIENCE (ICBECS), 2010 INTERNATIONAL CONFERENCE ON, IEEE, PISCATAWAY, NJ, USA, 23 April 2010 (2010-04-23), pages 1-4, XP031675214, ISBN: 978-1-4244-5315-3**
• **JI-ZHOU LI ET AL: "Estimation of longitudinal muscle motion using a primal-dual algorithm", BIOMEDICAL ENGINEERING INTERNATIONAL CONFERENCE (BMEICON), 2012, IEEE, 5 December 2012 (2012-12-05), pages 1-5, XP032334369, DOI: 10.1109/BMEICON.2012.6465425 ISBN: 978-1-4673-4890-4**

EP 2 988 675 B1

**Description**

The invention relates to a method and a device for quantitative dynamic evaluation of skeletal muscles functionality.

The performance of skeletal muscles depends on the ability of the same to develop a force and, for that reason, training is generally focused on muscle mass development. It is also true that the quality of muscle's action depends on many additional factors that are rarely assessed.

The first element involved in the quality of the development of strength concerns with the uniformity of the distribution of muscle tension along the anatomical structure, from bone insertion, through the tendons and along the muscle itself. It is thus important to be able to verify, from a dynamic point of view, the affective direction of stress and traction on the insertion points and how the muscle shortens according to its contractile ability. A muscle that presents, for example, a reinforced region connected to a less powerful one causes a local increase of the stresses in the areas where the region connects, with a corresponding increase of the potential risk of injury. These non-uniformities in the muscular function may develop as a result of a rehabilitation program or an athletic intense workload or for individual physiological characteristics of the athlete.

A second element of fundamental importance for the performance of the musculature relates to the presence or not of an adequate dynamic balance between the various muscular structures. Each action of the musculoskeletal system is driven by a number of muscles (agonists) that work together to perform the action in the most appropriate manner with the desired strength in the required time and along the correct direction in space and a corresponding number of muscles (antagonists) that are released so as not to oppose to the motion. This is what happens in every joint, the flexion of the hips, knees, ankles, as well as in the movements of the arms, hands and feet, even up to the toes, lumbar and cervical spine to mention only the main ones, or the opening and closing of the jaw.

In simplified terms, every action performed by a muscle element or elements (the agonists) involves a reaction of another element or elements (antagonists): the contraction of a muscle agonist is necessarily accompanied by the corresponding relaxation of the antagonist muscle. It is therefore evident the necessity of a balanced development between agonist and antagonist. A very common example in football, caused by normal training techniques, is the imbalance between the quadriceps (agonist of knee extension) and the flexors (antagonist). Excessively enhanced flexors restrain the action of the quadriceps and cause inaccuracy in the shooting besides causing overload on the knee with subsequent risk of muscle and tendon injuries.

It is therefore necessary to develop methods of training and preparation in football and in other disciplines, such as dance, as well as in therapeutic pathways including orthodontic ones, allowing a balanced development of the muscles. This balance is the essential objective in training and rehabilitation techniques.

The difficulties in the development of such balanced muscle training methods are mainly of a technical nature due to the lack of technology, simple and non-invasive, for a reproducible and objective measurement of the muscular capacity dynamics, i.e. during a muscular action, involving a single or multiple muscle elements at the same time. This technological deficiency has not allowed the development of case studies thus leaving the quality of the training completely in the hands of the individual trainer.

From document EP1297786 it is known how to use echographic acquisitions to evaluate the anatomic properties of muscle structures. This document goes in the right direction as it outlines how a simple and non-invasive methodology as the echographic one can be used to quantitative study muscle functionality. This study, however, is limited to the evaluation of the dilation and contraction in a region of a muscle by using spatio-temporal diagrams that provide the entity of the deformation along the longitudinal direction of the muscle. This allows measuring changes in the contraction of a muscle, for example as a function of different training sets, but it doesn't allow evaluating the uniformity of the muscle activity, as well as the synchronicity of the deformation between agonist and antagonist muscles, which represent indispensable aspects for the development of balanced training methodologies.

It is thus an object of the present invention to overcome, at least partially, these drawbacks.

The invention reaches the aim with a method for quantitative dynamic evaluation of skeletal muscles functionality according to claim 1.

In essence the invention is based on the study of the fields of the deformations of one or more muscle structures through analysis of sequences of ultrasound images acquired during the period of time in which such structures perform a predefined action.

The images can be related to a single muscle element or two muscle elements (agonist-antagonist), or more muscular elements involved in single action. Where with the term image is to be intended both a traditional two-dimensional image (2D) and a three-dimensional volumetric acquisition (3D). The sequences of images are typically synchronous between them or temporally staggered by a known amount in order to allow the comparison between the measured deformation fields.

According to an embodiment, the method provides to evaluate the uniformity of muscle activity within same muscles and/or different muscle elements involved in the same action and/or synchronicity in the deformation between agonist and antagonist muscles through comparison of the measurements in terms of intensity and/or timing of events.

Particularly, it can be provided the step of comparing the behaviour in time of the deformation of an agonist with the behaviour of the deformation in the corresponding antagonist muscle to detect situations of overload of the former with reference to the latter, or vice versa, for example, by measuring the time shift between a contraction curve of the agonist muscle and a relaxation curve of the corresponding antagonist muscle.

The evaluation of the muscle deformation is advantageously performed through "Optical Flow" or Particle Image Velocimetry" techniques (OF-PIV) as disclosed, for example, in Singh A. Optic Flow Computation: A Unified Perspective. Piscataway, NJ: IEEE Comput. Soc. Press, 1992, Barron JL, Fleet DJ, Beauchemin S. Performance of optical flow techniques. International Journal of Computer Vision 1994;12:43-77, Adrian RJ Twenty years of particle image velocimetry. Experiments in Fluids 2005; 39, 159-169. These methods of image analysis, known as " speckle tracking " or "feature tracking" in echocardiography, are widely used in cardiology to evaluate the performance of the heart muscle in his periodical contraction and relaxation as taught by the documents EP1520517 and US7343031.

Document EP2347716A1 discloses how the so-called Block Matching, a well-known PIV technique, can be used for evaluation of motion vector distribution in muscles. Horn and Schunk's optical flow algorithm is disclosed in Jun Shi et Al, Recognition of Finger Flexion from Ultrasound Image with Optical Flow: A Preliminary Study, Biomedical Engineering and Computer Science (ICBECS), 2010 Internation Conference on, IEEE, PISCATAWAY, NJ, USA, 23 April 2010, pages 1-4, while the primal-dual optical flow algorithm is disclosed in Ji-Zhou Li et Al., Estimation of longitudinal muscle motion using primal-dual algorithm, Biomedical Engineering International Conference (BMEICON), 2012, IEEE, 5 December 2012, pages 1-5.

The innovative element in the present invention is a review of these methods and the consequent adaptation for the evaluation of the dynamic properties of the different regions with different muscle characteristics during a movement with a predefined, not sinusoidal, temporal profile as well as in the comparative assessment of different elements of different muscle groups.

Particular importance is posed to the evaluation of the deformation and, in particular, the strain rate of skeletal muscles' elements, and thus to the uniformity and balance of muscular activity within the same muscles, as well as in the synchronicity of the deformation between agonist and antagonist muscles. In the above mentioned references only motion vector distribution is determined, i.e. instantaneous information of the movement. Deformations/strain rates are not instantaneous quantities, but integral quantities that have to be derived from motion vectors. Further processing of motion vectors is envisaged, for example in EP2347716A1, but it is limited to the calculation of several type of scalars (eigenvalues, divergence, rotation) not useful for determining measurements of deformations and/or strain rates which represent the key of the invention.

To this end, the method according to the invention involves calculating not simply the velocity of points in the image sequence, but relative motion, thus tissue deformation, for example by calculating the spatial variation of such velocities along points related by their brightness in subsequent images of the sequence or sequences of input images.

According to a particularly advantageous embodiment, the method provides for calculating the main directions of the deformations and the deformation values in such directions and showing the input images together with the vectors of the deformations. Such vectors differ from the velocity vector because they represent, through the shape of arrows, the orientation along which the deformation occurs, the principal eigenvectors of the deformation tensor, possibly with length proportional to said values of deformation in said directions.

The herein described methodology, based on the use of sequences of muscle images (movies) in multiple locations at the same time, possibly combined with procedures for the execution of repeatable actions, allows the assessment of the muscular balance, which is considered a key element in the latest training, physiotherapic, and physical correction techniques.

According to another aspect, the invention relates to a device according to claim 9.

The processing unit is configured to transform such sequence or sequences of images in sequences of measurements of deformations and/or strain rates in more spatial locations of the muscle or the muscles to evaluate performing one or more steps of the method according to the invention, particularly to determine the distribution of the vectors of the deformation and/or the synchronicity of the spatial evolution of the same.

Advantageously, the processing unit may be configured to evaluate the uniformity of muscle activity within same muscles and/or different muscle elements involved in the same action and/or the synchronicity of the deformation between agonist and antagonist muscles through comparison of the measurements in terms of intensity and/or synchronicity. Particularly the processing unit can be configured to calculate the main directions of the deformations and the deformation values in said directions. In this case the device can advantageously output the sequence or sequences of images together with the vectors of deformations, for example superimposing said vectors on the input images. The vectors typically have the shape of arrows oriented in the principal directions and length proportional to the values of deformation in said directions. This representation is particularly advantageous as it provides an immediate graphical indication of the behaviour of the deformations in time.

The device is typically provided for being interfaced with an echographic apparatus, the input sequences of images coming from said apparatus via data exchange on a mass memory or connection, wired or wireless, direct or through a network LAN, WAN or the Internet.

Advantageously the device can be provided for being interfaced, directly or through the echographic apparatus, with a system for the execution of a predefined, repeatable joint movement and/or a system for electro-induced stimulations capable of inducing predefined muscle contractions in one or more sites to allow acquisitions during reproducible movements for inter-individual and intra-individual comparisons at different times of training or rehabilitation.

Specifically the device can be provided in combination with an echographic apparatus having at least a probe for acquiring sequences of two-dimensional or three-dimensional echographic images of one or more muscles, there being provided means for synchronizing the acquisition in different zones of the same muscle or of different muscles.

The echographic apparatus can also be provided with at least two probes contemporaneously drivable to synchronously acquire sequences of two-dimensional or three-dimensional echographic images related to different muscles or different zones of the same muscle.

It is possible also to consider the combination of the device with at least two echographic apparatus having at least one probe for acquiring sequences of two-dimensional or three-dimensional echographic images of one or more muscles under investigation. In this case the sequences of images related to different muscles or different zones of the same muscle are acquired synchronously using means for synchronizing the acquisition of the two echographic apparatus, such as, for example, a protocol of communication of the two apparatus or a manual or automatic trigger, which, in its simplest configuration, is a switch manually actuated on both the apparatus.

According to a particularly advantageous embodiment, it is possible to consider the device in combination with a cinematically constrained machine, like those used in fitness or rehabilitation programmes as well as machines specifically used in other disciplines such as Gyrotonic or Pilates as well as GAIT analysis or with orthodontic appliances, and/or an electro-stimulation machine for muscle stimulation in coded and repeatable sequences. In this case the device may advantageously comprise an input for receiving such coded sequences through manual input or direct data exchange through an interface with the cinematically constrained machine or the electro-stimulation machine, the processing unit being sensitive to such input to perform comparisons between the deformations or strain rates as a function of the type of the stress induced by the machine to the muscle or muscles to evaluate.

According to another aspect, the invention relates to an echographic apparatus for evaluating the uniformity of the muscle activity within same muscles and/or different muscle elements involved in the same action and/or the synchronicity of deformation between agonist muscles and antagonist muscles. The apparatus comprises a device according to the invention capable to measure strain and/or strain rates from sequences obtained acquiring with one or more echographic probes one or more zones of the same muscle or of different muscles involved in the same action and operate a comparison between such measurements in terms of intensity and/or synchronicity.

According to an improvement, the apparatus may comprise at least two probes constrained among them for being placed on different zones of the same muscle and/or different muscles to contemporaneously acquire the related echographic images.

Advantageously the probe or the probes are supported by a device having a shape so as to be kept constrained with the anatomical element comprising the muscle to evaluate, such as, for example, a bandage, a collar, a brace or the like.

Further improvements of the invention will form the subject of the dependent claims.

The characteristics of the invention and the advantages derived therefrom will be more apparent from the following description of non-limiting embodiments, illustrated in the annexed drawings, in which:

Fig. 1 shows an ultrasonic image of a muscle region (10x100mm) at two instants during a muscle contraction. The arrow shows the muscle deformation through the relative displacement of two reference points recognizable in the two images and followed automatically in the sequence according to the method of the present invention;

Fig. 2 shows a space-time ultrasound image (M-mode) along a muscle (10 cm ) during the movement (5 seconds). This representation shows the spatial variation of the deformation moving from the proximal (1) to the distal region (2);

Fig. 3 shows an ultrasound image of a muscular tissue (left), and the evaluation of the strain rate field (indicated by the vectors) automatically calculated by the method and the device according to the invention. From this vector field is then possible to evaluate the properties of the deformation as shown by way of example in grayscale in the right image;

Figg. 4-7 illustrate an exemplified block diagram of some embodiments of the device according to the present invention .

The innovative technical element at the base of the present invention lends itself to multiple realizations of increasing complexity.

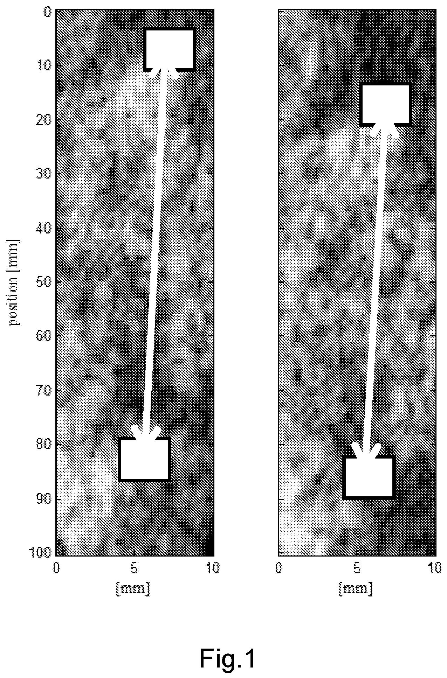

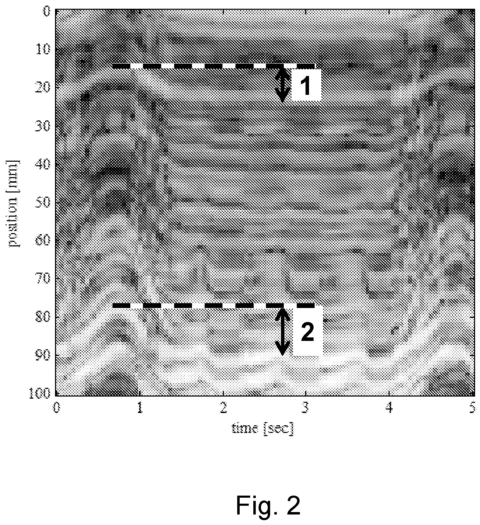

The common denominator of all these possible embodiments is represented by an image analysis device that, in its simplest form, is constituted by a processing unit capable of processing sequences of images generated by an ultrasound apparatus to provide sequences of measurements of spatial deformations such as those shown in Fig.1 and 2.

As already said, the objective evaluation of the muscle deformation can be advantageously performed with the already mentioned "Optical Flow" or "Particle Image Velocimetry" (OF-PIV) techniques adapted for the evaluation of dynamic properties of different regions with different muscular characteristics during a movement having a predetermined temporal profile, as well as the comparative evaluation of different elements of different muscle groups.

In essence, OF-PIV techniques are based on the concept of estimating motion of objects by comparing images taken at successive instants of time assuming that the brightness of each point of the original image moves rigidly in the images of the sequence. The images can be either two-dimensional three-dimensional. The following will, however, only deal with the two-dimensional case. Obviously, this should not be construed as limiting the scope of protection, but represents only an exemplification for an immediate grasp of the mathematical concepts used.

Indicating by B ( x , y , t ) the brightness of a point P of coordinates (x , y) at the time instant t, with Vx, Vy the velocity components, respectively along the x axis and along the y axis, it can be shown that the above assumption on B implies that the following equation should be satisfied:

$$\frac{\partial B}{\partial t} + V_x \frac{\partial B}{\partial x} + V_y \frac{\partial B}{\partial y} = 0$$

There are several ways to use this equation. The document Barron JL, Fleet DJ, Beauchemin S. Performance of optical flow techniques, International Journal of Computer Vision 1994; 12:43-77 makes an overview of these possible ways of resolution.

Although any resolution technique may be employed, the inventors have found that in the particular case of the detection of deformations muscle via echo images, the techniques of windowing and error minimization are well appropriate in terms of complexity of calculation, and thus processing speed, and accuracy of the results.

Specifically, the speed for each point of the image can be advantageously estimated by defining a window of pixels W and minimizing the quantity:

$$E = \sum_W \left( \frac{\partial B}{\partial t} + V_x \frac{\partial B}{\partial x} + V_y \frac{\partial B}{\partial y} \right)^2$$

Posing the derivative to zero, i.e.

$$\begin{cases} \dfrac{\partial E}{\partial V_x} = 0 \\ \dfrac{\partial E}{\partial V_y} = 0 \end{cases}$$

We obtain

$$\begin{cases} \sum_W \left( \dfrac{\partial B}{\partial t} + V_x \dfrac{\partial B}{\partial x} + V_y \dfrac{\partial B}{\partial y} \right) \dfrac{\partial B}{\partial x} = 0 \\ \sum_W \left( \dfrac{\partial B}{\partial t} + V_x \dfrac{\partial B}{\partial x} + V_y \dfrac{\partial B}{\partial y} \right) \dfrac{\partial B}{\partial y} = 0 \end{cases}$$

That provides the following linear system

$$\begin{bmatrix} \sum_W \dfrac{\partial B}{\partial x}\dfrac{\partial B}{\partial x} & \sum_W \dfrac{\partial B}{\partial x}\dfrac{\partial B}{\partial y} \\[2em] \sum_W \dfrac{\partial B}{\partial x}\dfrac{\partial B}{\partial y} & \sum_W \dfrac{\partial B}{\partial y}\dfrac{\partial B}{\partial y} \end{bmatrix} \cdot \begin{bmatrix} V_x \\[2em] V_y \end{bmatrix} = \begin{bmatrix} \sum_W \dfrac{\partial B}{\partial t}\dfrac{\partial B}{\partial x} \\[2em] \sum_W \dfrac{\partial B}{\partial t}\dfrac{\partial B}{\partial y} \end{bmatrix}$$

from which we can derive the unknowns Vx and Vy and, therefore, the field of velocities.

The deformation rates calculated by the method and the device according to an embodiment of the invention are represented by the changes of velocities in space i.e. by the gradient of the velocity vectors

$$\nabla \overline{V} = \begin{bmatrix} \dfrac{\partial V_x}{\partial x} & \dfrac{\partial V_x}{\partial y} \\[2em] \dfrac{\partial V_y}{\partial x} & \dfrac{\partial V_y}{\partial y} \end{bmatrix}$$

This matrix can be written as a sum of a symmetric matrix U and an antisymmetric matrix R in the following way

$$\nabla \overline{V} = \begin{bmatrix} \dfrac{\partial V_x}{\partial x} & \dfrac{1}{2}\left(\dfrac{\partial V_x}{\partial y}+\dfrac{\partial V_y}{\partial x}\right) \\[2em] \dfrac{1}{2}\left(\dfrac{\partial V_x}{\partial y}+\dfrac{\partial V_y}{\partial x}\right) & \dfrac{\partial V_y}{\partial y} \end{bmatrix} + \begin{bmatrix} 0 & \dfrac{1}{2}\left(\dfrac{\partial V_x}{\partial y}-\dfrac{\partial V_y}{\partial x}\right) \\[2em] -\dfrac{1}{2}\left(\dfrac{\partial V_x}{\partial y}-\dfrac{\partial V_y}{\partial x}\right) & 0 \end{bmatrix}$$

The symmetric matrix U is a pure deformation rate tensor, while the asymmetric matrix R a pure rigid rotation rate involving no deformation by itself.

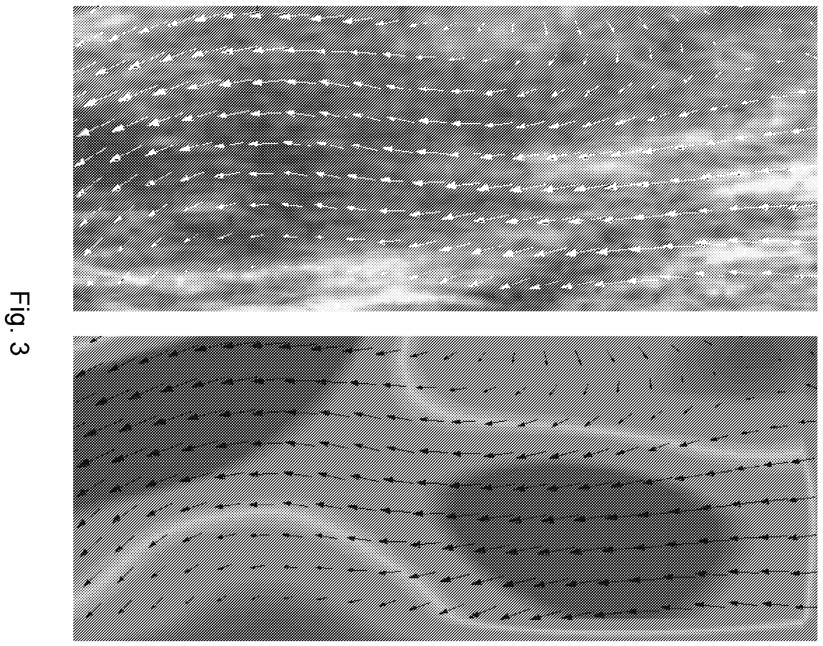

According to a particularly advantageous implementation form, the invention comprises the step to diagonalize the symmetric matrix to determine the lengthening / shortening (eigenvalues) along the principal directions (eigenvectors), i.e. the amount of pure deformation without shear. To such extent the method provides for calculating the principal directions of the deformations and the deformation values in such directions and showing the input images together with the vectors of the deformations. The vectors typically have the shape of arrows oriented in the principal directions and length proportional to the values of deformation in said directions as shown in fig. 3.

This is particularly advantageous because it allows for an immediate feedback, also visual, of the behaviour of the deformations of the muscles under examination in terms vectors. It is, in fact, not so much the tissue velocity but the strain fields, i.e. the knowledge of the distribution of the deformation in the 2D or 3D space contained in the images, that allows to make direct and immediate comparisons between parts of the same muscle or of different muscles in order to evaluate the uniformity of the muscle action, as well as the synchrony of the deformation, aspects necessary to develop innovative methods of training or rehabilitation able to analytically evaluate individual progress and the answers to specific activities, and to balance the development of different muscle groups, reducing the risk of injury due to an imbalance between agonist and antagonist muscles or to an intensification of efforts in limited regions.

The image analysis device of the present invention lends itself to many embodiments.

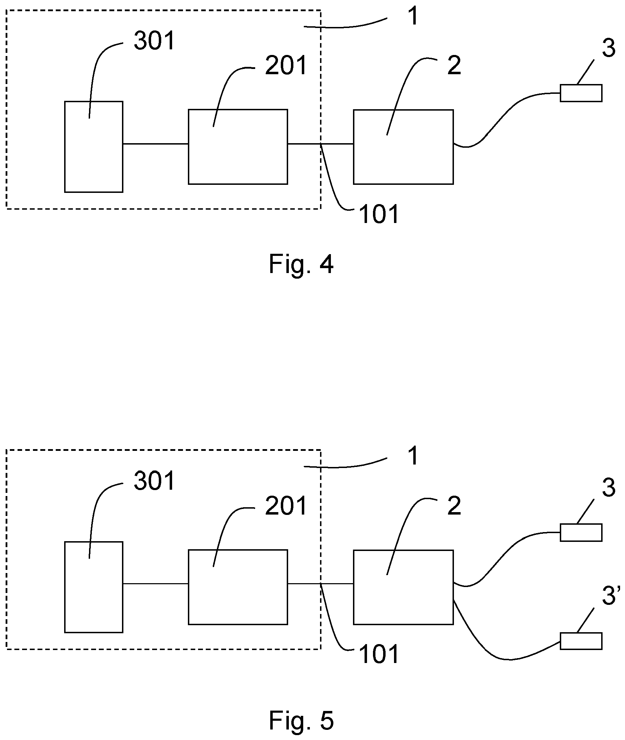

In its simplest form shown in Fig 4, the device 1 is provided in combination with an echographic apparatus 2 having a probe 3 capable of acquiring sequences of images. The apparatus 2 is connected in a physical manner or through wireless computer connections, to an input 101. It is also possible to provide that the exchange of data between apparatus 2 and device 1 is carried out through mass memories or in a way completely independent from the operation mode of the echographic apparatus 2 that, for this reason, can be of any type . The processing unit 201 of the device 1 reads the input sequences and process them to assess the spatial and temporal distribution of muscle deformation and show the results of the analysis on a monitor 301 in graphics and/or numeric form. The processing unit 201 may be a dedicated microprocessor system or, more generally, a PC also of the general purpose type. The characteristics of the unity 201 will obviously reflect on the processing speed.

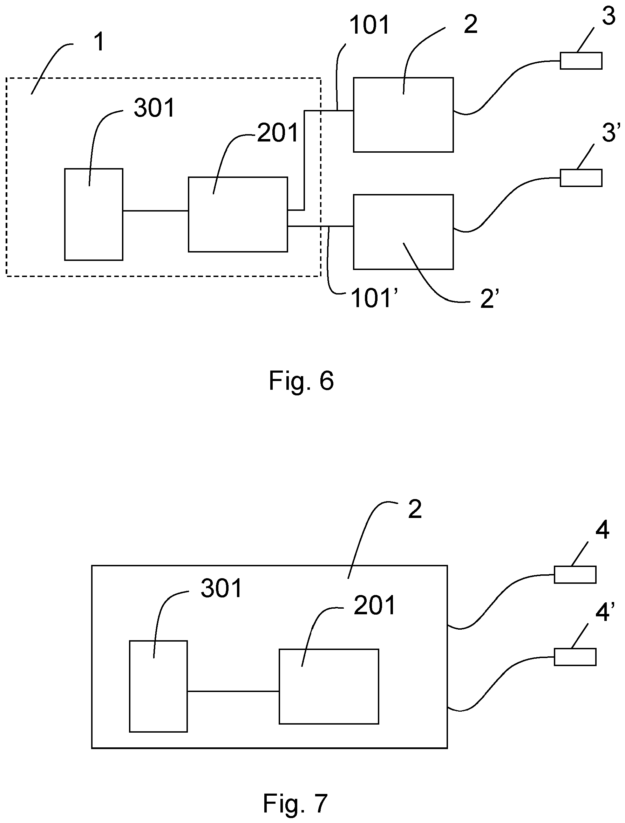

A more sophisticated embodiment, shown in Fig 5, comprises two or more ultrasound probes 3, 3' connected to the same echographic apparatus 2 for the simultaneous acquisition on two or more muscle groups so that the device 1 can perform a comparative evaluation of the relative deformations. In this case it is necessary that the apparatus used is capable of handling two or more probes simultaneously or multiplexed so as to ensure temporally staggered acquisitions of a known quantity. It is also possible to envisage that the device 1 is interfaced with two or more echographic apparatus 2, 2' as shown in Fig. 6. This allows the use of conventional echographic apparatus, leaving, for example, to the user the responsibility for checking the acquisition times for example by simultaneously pressing the freeze of the two apparatus or by connecting in parallel a pedal that acts on the freeze key of each echographic apparatus.

This solution is definitely more flexible in terms of use of ultrasound systems, but certainly not the best in terms of effectiveness of the device.

The optimum solution is, in fact, the one that allows the integration of the device according to the invention within an echographic apparatus as schematically shown in Fig 7. The device 1, in this case, may be part of the processing system of the ultrasound images with the monitor 301 that coincides with the same main monitor of the apparatus, thus creating a very compact system dedicated to the analysis of muscle deformations.

As far as ultrasound probes 4 are concerned, these can be of the traditional type or built ad hoc, for example to be integrated within a band to be placed around a leg for the analysis of the deformation of the leg muscles, or a collar or a bust for analyzing other anatomic regions.

According to a particularly advantageous implementation form, the device according to the invention is provided in combination with an auxiliary mechanical system to simplify the execution of predefined movements in a systematic and reproducible way, or to an electro-stimulation machine capable of inducing predetermined muscle contractions in one or multiple sites. In this way it is possible to perform reproducible acquisitions during reproducible movements for inter-individual and intra -individual comparisons at different times of training or rehabilitation. Such systems may be constrained kinematic machines like, for examples, those used in fitness or rehabilitation programmes, as well as specific machines of other disciplines such as, for example, the Gyrotonic or Pilates, or the equipment for GAIT analysis, or specific orthodontic appliances, and/or an electro-stimulation machine for muscle stimulation in coded and repeatable sequences.

In this case the device 1 may advantageously comprise an input (not shown in figures) for receiving such coded sequences through manual input or direct data exchange through an interface with the cinematically constrained machine or the electro-stimulation machine so that the processing unit 201 could perform comparisons between the deformations or strain rates as a function of the type of the stress induced by the machine to the muscle or muscles to evaluate.

## Claims

1. A method for quantitative dynamic evaluation of skeletal muscle functionality, carried out on a processing unit (201), comprising the following steps:

   a) receiving one or more sequences of two-dimensional or three-dimensional echographic images of the muscle under investigation;
   b) transforming such sequence or sequences of images in sequences of measurements of deformations in more spatial locations of the muscle;
   c) outputting such sequences of spatial measurements in numeric and/or graphical format

   wherein the measurements of deformation are obtained using the following steps:

   - calculating the velocity of points or zones having the same brightness in subsequent images of the input sequence or sequences of images;
   - calculating the spatial gradient matrix of said velocities;
   - dividing the spatial gradient matrix in a symmetrical matrix of pure deformation and in an asymmetrical matrix of pure rotation;
   - diagonalising the symmetrical matrix with calculation of the related eigenvalues;
   - calculating the corresponding eigenvectors;
   - showing, overlapped on the input images, arrows oriented in the directions of the eigenvectors, indicating the directions of the deformations, and with length proportional to the eigenvalues, indicating the values of deformations.

2. Method according to claim 1, wherein step b) comprises determining the distribution of the vectors of the deformation and/or the synchronicity of the spatial evolution of the same.

3. Method according to claim 1 or 2, wherein step a) comprises receiving sequences of images related to different muscles and/or different zones of the same muscle.

4. Method according to claim 3, wherein the sequences of images are substantially synchronous or temporally shifted of a known quantity.

5. Method according to one or more of the preceding claims, **characterised in** comprising the step of evaluating the uniformity of muscle activity within same muscles and/or different muscle elements involved in the same action and/or the synchronicity of the deformation between agonist and antagonist muscles through comparison of the measurements in terms of intensity and/or synchronicity.

6. Method according to one or more of the preceding claims, **characterised in** comprising the step of comparing the behaviour of the deformation of an agonist muscle with the behaviour of deformation of the correspondent antagonist muscle to detect situations of overload of the former with reference to the latter or vice versa.

7. Method according to claim 6, wherein a contraction curve of the agonist muscle and a relaxation curve of the corresponding antagonist muscle are shown, the situations of overload being detected by measuring the temporal shift between such curves.

8. Method according to one or more of the preceding claims, **characterised in** comprising the step of calculating the main directions of the deformations and the deformation values in such directions and showing the input images together with the vectors of the deformations.

9. Device for quantitative dynamic evaluation of skeletal muscle functionality, comprising:

   a) an input (101) for receiving one or more sequences of two-dimensional or three-dimensional echographic images of the muscle or the muscles under investigation;
   b) a processing unit (201) for transforming such sequence or sequences of images in sequences of spatial measurements;
   c) d) an output (301) for outputting such sequences of spatial measurements in numeric and/or graphical format,

   wherein the processing unit (201) is configured to transform such sequence or sequences of images in sequences of measurements of deformations in more spatial locations of the muscle performing the method according to one or more of the preceding claims.

10. Device according to claim 9, wherein said processing unit (201) is configured to determine the distribution of the vectors of the deformation and/or the synchronicity of the spatial evolution of the same.

11. Device according to claim 9 or 10, wherein the processing unit (201) is configured to evaluate the uniformity of muscle activity within same muscles and/or different muscle elements involved in the same action and/or the synchronicity of the deformation between agonist and antagonist muscles through comparison of the measurements in terms of intensity and/or synchronicity.

12. Device according to any preceding claim 9 to 11, wherein the processing unit (201) is configured to calculate the main directions of the deformations and the deformation values in said directions, the device outputting the sequence or sequences of images together with the vectors of deformations.

13. Device according to any preceding claim 9 to 12, **characterised in that** it is provided for being interfaced with an echographic apparatus (2), the input sequences of images coming from said apparatus via data exchange on a mass memory or connection, wired or wireless, direct or through a network LAN, WAN or the Internet.

14. Device according to any preceding claim 9 to 13, **characterised in that** it is provided for being interfaced, directly or through the echographic apparatus (2), with a system for the execution of a predefined, repeatable joint movement and/or a system for electro-induced stimulations capable of inducing predefined muscle contractions in one or more sites.

**15.** Device according to any preceding claim 9 to 14, **characterised in that** it is provided in combination with an echographic apparatus (2) having at least a probe (3) for acquiring sequences of two-dimensional or three-dimensional echographic images of one or more muscles, there being provided means for synchronising the acquisition in different zones of the same muscle or of different muscles.

**16.** Device according to any preceding claim 9 to 15, **characterised in that** it is provided in combination with an echographic apparatus (2) having at least two probes (3, 3') contemporaneously drivable to synchronously acquire sequences of two-dimensional or three-dimensional echographic images related to different muscles or different zones of the same muscle.

**17.** Device according to any preceding claim 9 to 16, **characterised in that** it is provided in combination with at least two echographic apparatus (2, 2') having at least one probe (3, 3') for acquiring sequences of two-dimensional or three-dimensional echographic images of one or more muscles under investigation, there being provided means for synchronising the acquisition of the two echographic apparatus to allow sequences of images related to different muscles or different zones of the same muscle to be acquired synchronously.

**18.** Device according to any preceding claim 9 to 17, **characterised in that** it is provided in combination with a cinematically constrained machine and/or a machine for muscular activity and/or an electro-stimulation machine for muscle stimulation in coded and repeatable sequences, the device comprising an input for receiving such coded sequences through manual input or direct data exchange through an interface with the machine, the processing unit being sensitive to such input to perform comparisons between the deformations as a function of the type of the stress induced by the machine to the muscle or muscles under evaluation.

**19.** Echographic apparatus for evaluating the uniformity of the muscle activity within same muscles and/or different muscle elements involved in the same action and/or the synchronicity of deformation between agonist and antagonist muscles, **characterised in** comprising a device (1) according to one or more of the preceding claims 9 to 18 capable to measure deformations from sequences obtained acquiring with one or more echographic probes (3) one or more zones of the same muscle or of different muscles involved in the same action and operate a comparison between such measurements in terms of intensity and/or synchronicity.

**20.** Echographic apparatus according to claim 19, **characterised in** comprising at least two probes (3, 3') constrained among them for being placed on different zones of the same muscle and/or different muscles to contemporaneously acquire the related echographic images.

**21.** Echographic apparatus according to claim 19 or 20, **characterised in that** the probe or the probes (3, 3') are supported by a device having a shape so as to be kept constrained with the anatomical element comprising the muscle under evaluation.

**22.** Echographic apparatus according to claim 21, **characterised in that** such device has the shape of a bandage, a collar, a brace or the like.

**Patentansprüche**

**1.** Verfahren zur quantitativen dynamischen Bewertung der Skelettmuskelfunktionalität, das auf einer Verarbeitungseinheit (201) durchgeführt wird und die folgenden Schritte umfasst:

a) Empfangen einer oder mehrerer Sequenzen von zweidimensionalen oder dreidimensionalen echographischen Bildern des untersuchten Muskels;
b) Umwandeln einer solchen Sequenz oder solcher Sequenzen von Bildern in Sequenzen von Messungen von Verformungen an mehreren räumlichen Stellen des Muskels;
c) Ausgabe solcher Sequenzen von räumlichen Messungen in numerischem und/oder grafischem Format

wobei die Messungen der Verformung durch die folgenden Schritte erhalten werden:

- Berechnen der Geschwindigkeit von Punkten oder Zonen mit gleicher Helligkeit in nachfolgenden Bildern der Eingangssequenz oder -sequenzen von Bildern;
- Berechnen der räumlichen Gradientenmatrix der genannten Geschwindigkeiten;

- Aufteilen der räumlichen Gradientenmatrix in eine symmetrische Matrix der reinen Verformung und in eine asymmetrische Matrix der reinen Drehung;
- Diagonalisieren der symmetrischen Matrix mit Berechnung der zugehörigen Eigenwerte;
- Berechnen der entsprechenden Eigenvektoren;
- Zeigen, überlagert auf den Eingangsbildern, von Pfeilen, die in den Richtungen der Eigenvektoren ausgerichtet sind, die Richtungen der Verformungen anzeigen, und mit einer Länge proportional zu den Eigenwerten, die die Werte der Verformungen anzeigen.

2. Verfahren nach Anspruch 1, wobei Schritt b) die Bestimmung der Verteilung der Vektoren der Verformung und/oder der Synchronität der räumlichen Entwicklung derselben umfasst.

3. Verfahren nach Anspruch 1 oder 2, wobei Schritt a) den Empfang von Sequenzen von Bildern umfasst, die sich auf verschiedene Muskeln und/oder verschiedene Zonen desselben Muskels beziehen.

4. Verfahren nach Anspruch 3, wobei die Sequenzen von Bildern im Wesentlichen synchron oder um eine bekannte Größe zeitlich verschoben sind.

5. Verfahren nach einem oder mehreren der vorhergehenden Ansprüche, **dadurch gekennzeichnet, dass** es den Schritt umfasst, die Gleichmäßigkeit der Muskelaktivität innerhalb derselben Muskeln und/oder verschiedener Muskelelemente, die an derselben Aktion beteiligt sind, und/oder die Synchronität der Verformung zwischen Agonisten- und Antagonisten-Muskeln durch Vergleich der Messungen in Bezug auf Intensität und/oder Synchronität zu bewerten.

6. Verfahren nach einem oder mehreren der vorhergehenden Ansprüche, **dadurch gekennzeichnet, dass** es den Schritt des Vergleichens des Verformungsverhaltens eines Agonisten-Muskels mit dem Verformungsverhalten des entsprechenden Antagonisten-Muskels umfasst, um Situationen der Überlastung des Ersteren in Bezug auf den Letzteren oder umgekehrt zu erkennen.

7. Verfahren nach Anspruch 6, wobei eine Kontraktionskurve des Agonisten-Muskels und eine Entspannungskurve des entsprechenden Antagonisten-Muskels gezeigt werden, wobei die Situationen der Überlastung durch Messung der zeitlichen Verschiebung zwischen diesen Kurven erfasst werden.

8. Verfahren nach einem oder mehreren der vorhergehenden Ansprüche, **dadurch gekennzeichnet, dass** es den Schritt der Berechnung der Hauptrichtungen der Verformungen und der Verformungswerte in diesen Richtungen und das Zeigen der Eingangsbilder zusammen mit den Vektoren der Verformungen umfasst.

9. Vorrichtung zur quantitativen dynamischen Bewertung der Skelettmuskelfunktionalität, umfassend:

a) einen Eingang (101) zum Empfangen einer oder mehrerer Sequenzen von zweidimensionalen oder dreidimensionalen echographischen Bildern des oder der untersuchten Muskeln;
b) eine Verarbeitungseinheit (201) zur Umwandlung einer solchen Sequenz oder solcher Sequenzen von Bildern in Sequenzen von räumlichen Messungen;
c) einen Ausgang (301) zur Ausgabe solcher Sequenzen von räumlichen Messungen in numerischem und/oder grafischem Format,

wobei die Verarbeitungseinheit (201) so ausgelegt ist, dass sie eine solche Sequenz oder Sequenzen von Bildern in Sequenzen von Messungen von Verformungen an mehreren räumlichen Stellen des Muskels umwandelt, die das Verfahren nach einem oder mehreren der vorhergehenden Ansprüche durchführen.

10. Vorrichtung nach Anspruch 9, wobei die Verarbeitungseinheit (201) so ausgelegt ist, dass sie die Verteilung der Vektoren der Verformung und/oder die Synchronität der räumlichen Entwicklung derselben bestimmt.

11. Vorrichtung nach Anspruch 9 oder 10, wobei die Verarbeitungseinheit (201) so ausgelegt ist, dass sie die Gleichmäßigkeit der Muskelaktivität innerhalb derselben Muskeln und/oder verschiedener Muskelelemente, die an derselben Aktion beteiligt sind, und/oder die Synchronität der Verformung zwischen Agonisten- und Antagonisten-Muskeln durch Vergleich der Messungen in Bezug auf Intensität und/oder Synchronität bewertet.

12. Vorrichtung nach einem der vorhergehenden Ansprüche 9 bis 11, wobei die Verarbeitungseinheit (201) so ausgelegt ist, dass sie die Hauptrichtungen der Verformungen und die Verformungswerte in diesen Richtungen berechnet, wobei die Vorrichtung die Sequenz oder Sequenzen von Bildern zusammen mit den Vektoren der Verformungen ausgibt.

13. Vorrichtung nach einem der vorhergehenden Ansprüche 9 bis 12, **dadurch gekennzeichnet, dass** sie dazu bereitgestellt ist, mit einem echographischen Gerät (2) verbunden zu werden, wobei die Eingangssequenzen von Bildern von diesem Gerät über einen Datenaustausch auf einem Massenspeicher oder einer Verbindung, verdrahtet oder drahtlos, direkt oder über ein Netzwerk-LAN, -WAN oder das Internet, kommen.

14. Vorrichtung nach einem der vorhergehenden Ansprüche 9 bis 13, **dadurch gekennzeichnet, dass** sie dazu bereitgestellt ist, direkt oder über das echographische Gerät (2) mit einem System zur Ausführung einer vordefinierten, wiederholbaren Gelenkbewegung und/oder einem System für elektroinduzierte Stimulationen, das in der Lage ist, vordefinierte Muskelkontraktionen an einer oder mehreren Stellen zu induzieren, verbunden zu werden.

15. Vorrichtung nach einem der vorhergehenden Ansprüche 9 bis 14, **dadurch gekennzeichnet, dass** sie in Kombination mit einem echographischen Gerät (2) mit wenigstens einer Sonde (3) zur Aufnahme von Sequenzen zweidimensionaler oder dreidimensionaler echographischer Bilder eines oder mehrerer Muskeln bereitgestellt ist, wobei Einrichtung zur Synchronisierung der Aufnahme in verschiedenen Zonen desselben Muskels oder verschiedener Muskeln bereitgestellt sind.

16. Vorrichtung nach einem der vorhergehenden Ansprüche 9 bis 15, **dadurch gekennzeichnet, dass** sie in Kombination mit einem echographischen Gerät (2) bereitgestellt ist, das wenigstens zwei Sonden (3, 3') aufweist, die gleichzeitig ansteuerbar sind, um synchron Sequenzen von zweidimensionalen oder dreidimensionalen echographischen Bildern aufzunehmen, die sich auf verschiedene Muskeln oder verschiedene Zonen desselben Muskels beziehen.

17. Vorrichtung nach einem der vorhergehenden Ansprüche 9 bis 16, **dadurch gekennzeichnet, dass** sie in Kombination mit wenigstens zwei echographischen Geräten (2, 2') mit wenigstens einer Sonde (3, 3') zur Aufnahme von Sequenzen von zweidimensionalen oder dreidimensionalen echographischen Bildern eines oder mehrerer untersuchten Muskeln bereitgestellt ist, wobei Einrichtung zur Synchronisierung der Aufnahme der beiden echographischen Geräte bereitgestellt ist, um die synchrone Aufnahme von Sequenzen von Bildern zu ermöglichen, die sich auf verschiedene Muskeln oder verschiedene Zonen desselben Muskels beziehen.

18. Vorrichtung nach einem der vorhergehenden Ansprüche 9 bis 17, **dadurch gekennzeichnet, dass** sie in Kombination mit einer Maschine mit kinematischem Zwang und/oder einer Maschine für Muskelaktivität und/oder einer Elektrostimulationsmaschine zur Muskelstimulation in kodierten und wiederholbaren Sequenzen bereitgestellt ist, wobei die Vorrichtung einen Eingang zum Empfangen solcher kodierten Sequenzen durch manuellen Eingang oder direkten Datenaustausch über eine Schnittstelle mit der Maschine umfasst, wobei die Verarbeitungseinheit für solchen Eingang empfindlich ist, um Vergleiche zwischen den Verformungen in Abhängigkeit von der Art der durch die Maschine auf den oder die bewerteten Muskeln ausgeübten Belastung durchzuführen.

19. Echographisches Gerät zur Bewertung der Gleichförmigkeit der Muskelaktivität innerhalb gleicher Muskeln und/oder verschiedener Muskelelemente, die an der gleichen Aktion beteiligt sind, und/oder der Synchronität der Verformung zwischen Agonisten- und Antagonisten-Muskeln, **dadurch gekennzeichnet, dass** es eine Vorrichtung (1) nach einem oder mehreren der vorhergehenden Ansprüche 9 bis 18 umfasst, die in der Lage ist, Verformungen aus erhaltenen Sequenzen zu messen, die mit einer oder mehreren echographischen Sonden (3) eine oder mehrere Zonen des gleichen Muskels oder verschiedener Muskeln, die an der gleichen Aktion beteiligt sind, erfassen, und einen Vergleich zwischen solchen Messungen in Bezug auf die Intensität und/oder Synchronität durchzuführen.

20. Echographisches Gerät nach Anspruch 19, **dadurch gekennzeichnet, dass** es wenigstens zwei Sonden (3, 3') umfasst, die untereinander gezwungen sind, um auf verschiedenen Zonen desselben Muskels und/oder verschiedener Muskeln platziert zu werden, um gleichzeitig die entsprechenden echographischen Bilder aufzunehmen.

21. Echographisches Gerät nach Anspruch 19 oder 20, **dadurch gekennzeichnet, dass** die Sonde oder die Sonden (3, 3') von einer Vorrichtung getragen werden, die so geformt ist, dass sie mit dem anatomischen Element, das den bewerteten Muskel umfasst, unter Zwang gehalten wird.

22. Echographisches Gerät nach Anspruch 21, **dadurch gekennzeichnet, dass** die Vorrichtung die Form einer Bandage, eines Halses, einer Klammer oder dergleichen hat.

**Revendications**

1. Procédé d'évaluation dynamique quantitative de la fonctionnalité des muscles squelettiques, réalisé sur une unité de traitement (201), comprenant les étapes suivantes :

   a) recevoir une ou plusieurs séquences d'images échographiques bidimensionnelles ou tridimensionnelles du muscle en examen ;
   b) transformer cette séquence ou ces séquences d'images en séquences de mesures de déformations en plusieurs emplacements du muscle ;
   c) sortir ces séquences de mesures spatiales sous format numérique et/ou graphique dans lequel les mesures de déformation sont obtenues en suivant les étapes suivantes :

   - calculer la vitesse des points ou des zones ayant la même luminosité dans les images suivantes de la ou des séquences d'images d'entrée ; calculer la matrice du gradient spatial desdites vitesses ;
   - diviser la matrice du gradient spatial en une matrice symétrique de déformation pure et en une matrice asymétrique de rotation pure ; diagonaliser la matrice symétrique et avec calcul des valeurs propres correspondantes ;
   - calculer les vecteurs propres correspondants ;
   - montrer, superposées aux images d'entrée, des flèches orientées dans les directions des vecteurs propres, indiquant les directions des déformations, et d'une longueur proportionnelle aux valeurs propres, indiquant les valeurs des déformations.

2. Procédé selon la revendication 1, dans lequel l'étape b) comprend la détermination de la distribution des vecteurs de la déformation et/ou du synchronisme de l'évolution spatiale de celle-ci.

3. Procédé selon la revendication 1 ou 2, dans lequel l'étape a) comprend la réception de séquences d'images relatives à différents muscles et/ou différentes zones du même muscle.

4. Procédé selon la revendication 3, dans lequel les séquences d'images sont sensiblement synchrones ou décalées dans le temps d'une quantité connue.

5. Procédé selon une ou plusieurs des revendications précédentes, **caractérisé en ce qu'**il comprend l'étape consistant à évaluer l'uniformité de l'activité musculaire au sein des mêmes muscles et/ou des différents éléments musculaires impliqués dans la même action et/ou le synchronisme de la déformation entre les muscles agonistes et antagonistes par la comparaison des mesures en termes d'intensité et/ou de synchronisme.

6. Procédé selon l'une ou plusieurs des revendications précédentes, **caractérisé en ce qu'**il comprend l'étape consistant à comparer le comportement de la déformation d'un muscle agoniste avec le comportement de la déformation du muscle antagoniste correspondant pour détecter des situations de surcharge du premier par rapport au second ou vice versa.

7. Procédé selon la revendication 6, dans lequel une courbe de contraction du muscle agoniste et une courbe de relaxation du muscle antagoniste correspondant sont représentées, les situations de surcharge étant détectées en mesurant le décalage temporel entre ces courbes.

8. Procédé selon l'une ou plusieurs des revendications précédentes, **caractérisé en ce qu'**il comprend l'étape consistant à calculer les directions principales des déformations et les valeurs de déformation dans ces directions et à afficher les images d'entrée avec les vecteurs des déformations.

9. Dispositif d'évaluation dynamique quantitative de la fonctionnalité des muscles squelettiques, comprenant :

   a) une entrée (101) pour recevoir une ou plusieurs séquences d'images échographiques bidimensionnelles ou tridimensionnelles du ou des muscles en examen ;
   b) une unité de traitement (201) pour transformer cette séquence ou ces séquences d'images en séquences de mesures spatiales ;

c) une sortie (301) pour restituer ces séquences de mesures spatiales sous format numérique et/ou graphique,

dans lequel l'unité de traitement (201) est configurée pour transformer cette séquence ou ces séquences d'images en séquences de mesures de déformations dans plusieurs emplacements spatiaux du muscle mettant en oeuvre le procédé selon l'une ou plusieurs des revendications précédentes.

10. Dispositif selon la revendication 9, dans lequel ladite unité de traitement (201) est configurée pour déterminer la distribution des vecteurs de la déformation et/ou le synchronisme de l'évolution spatiale de celle-ci.

11. Dispositif selon la revendication 9 ou 10, dans lequel l'unité de traitement (201) est configurée pour évaluer l'uniformité de l'activité musculaire au sein des mêmes muscles et/ou des différents éléments musculaires impliqués dans la même action et/ou le synchronisme de la déformation entre les muscles agonistes et antagonistes par comparaison des mesures en termes d'intensité et/ou de synchronisme.

12. Dispositif selon une quelconque revendication précédente 9 à 11, dans lequel l'unité de traitement (201) est configurée pour calculer les directions principales des déformations et les valeurs de déformation dans lesdites directions, le dispositif délivrant la ou les séquences d'images avec les vecteurs de déformations.

13. Dispositif selon une quelconque revendication précédente 9 à 12, **caractérisé en ce qu'**il est prévu pour être interfacé avec un appareil échographique (2), les séquences d'images d'entrée provenant dudit appareil via un échange de données sur une mémoire de masse ou une connexion, filaire ou sans fil, directe ou par l'intermédiaire d'un réseau LAN, WAN ou Internet.

14. Dispositif selon une quelconque revendication précédente 9 à 13, **caractérisé en ce qu'**il est prévu pour être interfacé, directement ou par l'intermédiaire de l'appareil échographique (2), avec un système d'exécution d'un mouvement articulaire prédéfini et répétable et/ou un système de stimulations électro-induites capable d'induire des contractions musculaires prédéfinies dans un ou plusieurs sites.

15. Dispositif selon une quelconque revendication précédente 9 à 14, **caractérisé en ce qu'**il est prévu en combinaison avec un appareil échographique (2) comportant au moins une sonde (3) pour l'acquisition de séquences d'images échographiques bidimensionnelles ou tridimensionnelles d'un ou de plusieurs muscles, des moyens étant prévus pour synchroniser l'acquisition dans différentes zones du même muscle ou de muscles différents.

16. Dispositif selon une quelconque revendication précédente 9 à 15, **caractérisé en ce qu'**il est fourni en combinaison avec un appareil échographique (2) comportant au moins deux sondes (3, 3') pilotées simultanément pour acquérir de façon synchrone des séquences d'images échographiques bidimensionnelles ou tridimensionnelles relatives à différents muscles ou à différentes zones du même muscle.

17. Dispositif selon une quelconque revendication précédente 9 à 16, **caractérisé en ce qu'**il est fourni en combinaison avec au moins deux appareils échographiques (2, 2') comportant au moins une sonde (3, 3') pour l'acquisition de séquences d'images échographiques bidimensionnelles ou tridimensionnelles d'un ou de plusieurs muscles en examen, des moyens étant prévus pour synchroniser l'acquisition des deux appareils échographiques afin de permettre l'acquisition synchrone de séquences d'images relatives à différents muscles ou à différentes zones du même muscle.

18. Dispositif selon une quelconque revendication précédente 9 à 17, **caractérisé en ce qu'**il est fourni en combinaison avec une machine à contrainte cinématographique et/ou une machine pour l'activité musculaire et/ou une machine d'électrostimulation pour la stimulation musculaire en séquences codées et répétables, le dispositif comprenant une entrée pour recevoir de telles séquences codées par saisie manuelle ou échange direct de données par une interface avec la machine, l'unité de traitement étant sensible à une telle entrée pour effectuer des comparaisons entre les déformations en fonction du type de contrainte induite par la machine sur le muscle ou les muscles évalués.

19. Appareil échographique pour évaluer l'uniformité de l'activité musculaire au sein de mêmes muscles et/ou d'éléments musculaires différents impliqués dans une même action et/ou le synchronisme de déformation entre muscles agonistes et antagonistes, **caractérisé en ce qu'**il comprend un dispositif (1) selon une ou plusieurs des revendications précédentes 9 à 18 apte à mesurer des déformations à partir de séquences obtenues en acquérant avec une ou plusieurs sondes échographiques (3) une ou plusieurs zones du même muscle ou de muscles différents impliqués dans une même action et à opérer une comparaison entre ces mesures en termes d'intensité et/ou de synchronisme.

**20.** Appareil échographique selon la revendication 19, **caractérisé en ce qu'**il comprend au moins deux sondes (3, 3') contraintes entre elles pour être placées sur différentes zones du même muscle et/ou de muscles différents afin d'acquérir simultanément les images échographiques correspondantes.

**21.** Appareil échographique selon la revendication 19 ou 20, **caractérisé en ce que** la sonde ou les sondes (3, 3') sont supportées par un dispositif ayant une forme telle qu'il est maintenu contraint par l'élément anatomique comprenant le muscle à évaluer.

**22.** Appareil échographique selon la revendication 21, **caractérisé en ce que** ce dispositif a la forme d'un bandage, d'un collier, d'une attelle ou équivalent.

Fig.1

Fig. 2

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1297786 A
- EP 1520517 A
- US 7343031 B
- EP 2347716 A1

**Non-patent literature cited in the description**

- **SINGH A.** Optic Flow Computation: A Unified Perspective. IEEE Comput. Soc. Press, 1992
- **BARRON JL ; FLEET DJ ; BEAUCHEMIN S.** Performance of optical flow techniques. *International Journal of Computer Vision,* 1994, vol. 12, 43-77
- **ADRIAN RJ.** Twenty years of particle image velocimetry. *Experiments in Fluids,* 2005, vol. 39, 159-169
- **JUN SHI et al.** Recognition of Finger Flexion from Ultrasound Image with Optical Flow: A Preliminary Study. *Biomedical Engineering and Computer Science (ICBECS), 2010 Internation Conference on, IEEE, PISCATAWAY, NJ, USA,* 23 April 2010, 1-4
- Estimation of longitudinal muscle motion using primal-dual algorithm. **JI-ZHOU LI et al.** Biomedical Engineering International Conference (BMEICON). IEEE, 05 December 2012, 1-5